# FASCICULE DE BREVET EUROPEEN

(11) **EP 2 607 353 B1**
(45) Date de publication et mention de la délivrance du brevet: **27.08.2014**
(21) Numéro de dépôt: 12191902.1
(22) Date de dépôt: 09.11.2012
(51) Int. Cl.: C07D 223/16

(54) **"Nouveau procédé de synthèse de l'ivabradine et de ses sels d'addition à un acide pharmaceutiquement acceptable"**
Neues Syntheseverfahren von Ivabradin und seinen Salzen als Zusatz zu einer pharmazeutisch akzeptierbaren Säure
New method for synthesising ivabradine and its added salts with a pharmaceutically acceptable acid.

(30) Priorité: 20.12.2011 FR 1103933
(43) Date de publication de la demande: 26.06.2013
(73) Titulaire: Les Laboratoires Servier, 92284 Suresnes Cedex (FR)
(72) Inventeur: Renaud, Jean-Luc, 14112 Bieville-Beuville (FR); Pannetier, Nicolas, 14000 Caen (FR); Lecouve, Jean-Pierre, 76600 Le Havre (FR); Vaysse-Ludot, Lucile, 76490 Saint-Wandrille-Rancon (FR); Moulin, Solenne, 22000 Saint-Brieuc (FR)

(56) Documents cités:
- EP-A1- 2 036 892
- EP-A1- 2 202 225
- WO-A1-2005/110993
- BHOR ET AL: "Direct reductive amination of carbonyl compounds with primary/secondary amines using recyclable water-soluble Fe<II>/EDTA complex as catalyst", TETRAHEDRON LETTERS, ELSEVIER, AMSTERDAM, NL, vol. 49, no. 6, 8 décembre 2007 (2007-12-08), pages 965-969, XP022417894, ISSN: 0040-4039, DOI: 10.1016/J.TETLET.2007.12.023
- S. ENTHALER: "Synthesis of Secondary Amines by Iron- Catalysed Reductive Amination", CHEMCATCHEM, vol. 2, 2010, pages 1411-1415, XP002671676, DOI: 10.1002/cctc.201000180
- T.C.JOHNSON, G,J.CLARKSON AND M. WILLS: "(Cyclopentadienone)iron Shvo Complexes: Synthesis and Applications to Hydrogen Transfer Reactions", ORGANOMETALLICS, vol. 30, 9 mars 2011 (2011-03-09), pages 1859-1868, XP002671677,

## Description

La présente invention concerne un procédé de synthèse de l'ivabradine de formule (I) : 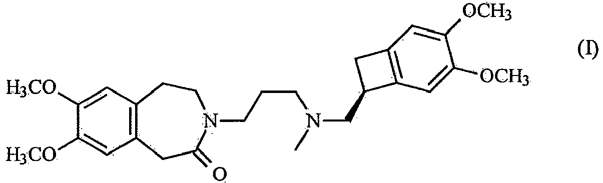 ou 3-{3-[{[(7S)-3,4-diméthoxybicyclo[4.2.0]octa-1,3,5-trién-7-yl]méthyl}(méthyl)amino] propyl}-7,8-diméthoxy-l,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859. Malheureusement, la voie de synthèse de l'ivabradine décrite dans ce brevet ne conduit au produit attendu qu'avec un rendement de 1%.

Une autre voie de synthèse de l'ivabradine, basée sur une réaction d'amination réductrice, a été décrite dans le brevet européen EP 1 589 005.

L'amination réductrice est une voie d'accès privilégiée pour préparer des amines. Dans la mesure où elle ne requiert pas d'isoler l'imine intermédiaire formée, cette réaction de couplage entre un aldéhyde et une amine en présence d'un agent de réduction est largement utilisée pour la synthèse de molécules d'intérêt dans le domaine pharmaceutique ou agrochimique ainsi qu'en science des matériaux.

Les protocoles expérimentaux classiquement mis en oeuvre pour réaliser une amination réductrice sont :
- soit l'utilisation de quantités stoechiométriques de donneurs d'hydrures tels que les borohydrures (NaBH₄, NaBH₃CN ou NaBH(OAc)₃),
- soit l'hydrogénation catalytique.

L'utilisation de donneurs d'hydrures génère de nombreux déchets et les réactifs en eux-mêmes sont toxiques.

Dans le cas de l'hydrogénation catalytique, l'agent de réduction est l'hydrogène moléculaire, ce qui présente un intérêt écologique certain. La synthèse décrite dans le brevet EP 1 589 005 suit cette deuxième voie.

En effet, le brevet EP 1 589 005 décrit la synthèse du chlorhydrate de l'ivabradine à partir du composé de formule (II) : 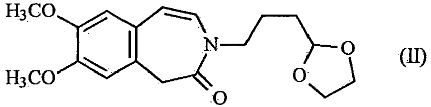 qui est soumis à une réaction d'hydrogénation catalytique en présence d'hydrogène et d'un catalyseur au palladium pour conduire au composé de formule (III) : 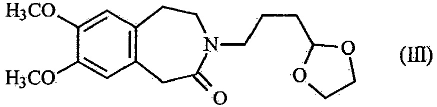 qui, sans être isolé, est mis en réaction, en présence d'hydrogène et d'un catalyseur au palladium, avec le composé de formule (IV) : 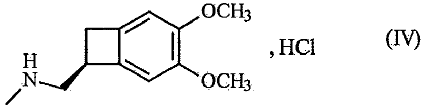 pour conduire à l'ivabradine de formule (I), sous forme de chlorhydrate.

L'inconvénient de cette voie de synthèse est d'utiliser un catalyseur au palladium.

Le palladium, tout comme le rhodium, le ruthénium ou l'iridium, métaux également utilisés pour catalyser les réactions d'amination réductrice, est un métal précieux dont la disponibilité faible, et par conséquent le prix élevé, ainsi que la toxicité limitent l'intérêt.

La présente demande décrit une voie de synthèse de l'ivabradine qui permet de s'affranchir de l'utilisation d'un métal précieux.

En effet, la présente invention concerne un procédé de synthèse de l'ivabradine de formule
(I): 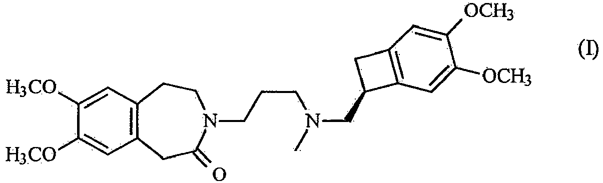 caractérisé en ce que l'on soumet le composé de formule (V) : 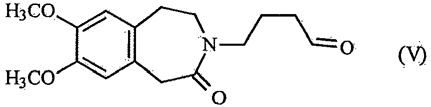 à une réaction d'amination réductrice par l'amine de formule (VI) : 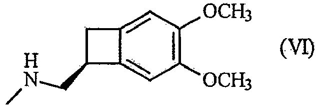 en présence d'un catalyseur à base de fer de formule (VII) : 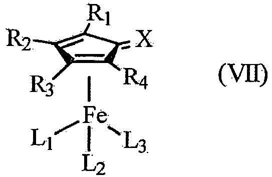 dans laquelle R₁, R₂, R₃ et R₄ représentent de manière indépendante :
   - un atome d'hydrogène, ou
   - un groupement -SiR₅R₆R₇, dans le quel R₅, R₆ et R₇, représentent de manière indépendante un groupement (C₁-C₆)alkyle linéaire ou ramifié, optionnellement substitué, ou un groupement aromatique ou hétéroaromatique optionnellement substitué, ou
   - un groupement aromatique ou hétéroaromatique, optionnellement substitué, ou
   - un groupement (C₁-C₆)alkyle linéaire ou ramifié, optionnellement substitué, ou
   - un groupement électroattracteur, ou
   - une aminé aliphatique, aromatique, hétéroaromatique ou portant un groupement électroattracteur, ou
   - un éther aliphatique, aromatique ou hétéroaromatique,
ou R₁ et R₂, ou R₂ et R₃, ou R₃ et R₄ forment deux à deux avec les atomes de carbone qui les portent un carbocycle ou un hétérocycle possédant 3 à 7 chaînons,

X représente :
- un atome d'oxygène, ou
- un groupement -NH, ou un atome d'azote substitué par un groupement aliphatique, aromatique, hétéroaromatique ou électroattracteur, ou
- un groupement -PH, ou un atome de phosphore substitué par un ou plusieurs groupements aliphatiques, aromatiques ou électroattracteurs, formant préférentiellement un groupement phosphine, phosphite, phosphonite, phosphoramidite, phosphinite, phospholane ou phospholène, ou
- un atome de soufre,

L₁, L₂ et L₃ représentent de manière indépendante un groupement carbonyle, nitrile, isonitrile, hétéroaromatique, phosphine, phosphite, phosphonite, phosphoramidite, phosphinite, phospholane, phospholène, amine aliphatique, amine aromatique, amine hétéroaromatique, amine portant un groupement électroattracteur, éther aliphatique, éther aromatique, éther hétéroaromatique, sulfone, sulfoxyde, sulfoximine, ou un carbène *N*-hétérocyclique possédant l'une des deux formules suivantes : 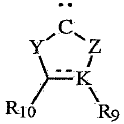 dans laquelle Y et Z représentent de manière indépendante un atome de soufre ou d'oxygène, ou un groupement NR₈, dans lequel R₈ représente un groupement alkyle optionnellement substitué, un groupement aromatique ou hétéroaromatique optionnellement substitué,
K représente un atome de carbone ou d'azote,
R₉, R₁₀ représentent de manière indépendante un atome d'hydrogène, un groupement alkyle optionnellement substitué, un groupement aromatique ou hétéroaromatique optionnellement substitué, un atome d'halogène, un éther aliphatique, aromatique ou hétéroaromatique, une amine aliphatique aromatique ou hétéroaromatique,
ou R₉ et R₁₀ forment deux à deux avec les atomes qui les portent un carbocycle ou un hétérocycle possédant 3 à 7 chaînons,
ou 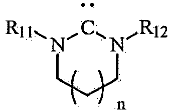 dans laquelle R₁₁ et R₁₂ représentent de manière indépendante un groupement alkyle optionnellement substitué, un groupement aromatique ou hétéroaromatique optionnellement substitué et n est égal à 1 ou 2,
en présence ou non de *N*-oxyde de triméthylamine,
sous pression de dihydrogène,
dans un solvant organique ou un mélange de solvants organiques.

Contrairement aux dérivés de métaux précieux, ceux du fer sont en général non toxiques et les sels de fer sont présents en abondance dans la nature. Ce sont des espèces métalliques non nocives pour l'environnement. La présente invention propose l'utilisation de complexes du fer qui sont en outre faciles à manipuler.

Les complexes de fer sont connus pour catalyser des réactions d'amination réductrice en présence d'hydrures silylés (Enthaler S. ChemCatChem 2010, 2, 1411-1415) mais seuls deux exemples dans la littérature décrivent une amination réductrice basée sur une hydrogénation catalytique (Bhanage B. M. et al Tet. Lett. 2008, 49, 965-969 ; Beller M. et al. Chem Asian J. 2011, 6, 2240-2245).

Les conditions opératoires décrites par Bhanage requièrent la présence d'un complexe hydrosoluble composé d'un sel de fer(II) et d'EDTA dans un milieu porté à haute température et haute pression d'hydrogène.

L'application des conditions opératoires décrites dans cette publication à la préparation de l'ivabradine n'a pas permis d'obtenir le produit attendu.

Le tableau suivant récapitule les essais effectués dans un autoclave de 20 mL en présence de 4mL d'eau dégazée, sous 30 bars d'hydrogène pendant 18h. Les réactions ont été réalisées à l'échelle de 0,5 mmol avec un rapport aldéhyde/amine de 1/1,5 (sauf ligne 1 : aldéhyde/amine (1/1) et addition d'1% d'acide p-toluène sulfonique). Les quantités en % molaire sont calculées par rapport à l'aldéhyde.

NTf est l'abbréviation pour trifluorométhanesulfonamide.

**Table 1. Résultats des réactions d'amination réductrice catalysée par les sels de fer (II)**

| | Sel de fer(II) (mol%) | Quantité (mol%) de EDTANa₂ | Température (°C) | Observations |
|---|---|---|---|---|
| **1** | FeSO₄.7 H₂O (2) | 10 | 150 | 0% d'ivabradine - dégradation |
| **2** | FeSO₄.7 H₂O (2) | 10 | 85 | 0% d'ivabradine |
| **3** | FeCl₂.4 H₂O (5) | 10 | 85 | 0% d'ivabradine |
| **4** | FeBr₂.4H₂O (5) | 10 | 85 | 0% d'ivabradine |
| **5** | Fe(BF₄)₂.6H₂O (5) | 10 | 85 | 0% d'ivabradine |
| **6** | Fe(NTf₂)₂.6H₂O (5) | 10 | 85 | 0% d'ivabradine |

La publication de Beller décrit l'amination réductrice de divers aldéhydes par des dérivés d'aniline en présence de Fe₃(CO)₁₂ dans le toluène sous 50 bars d'hydrogène à 65°C.

L'application des conditions décrites dans cette publication à la préparation de l'ivabradine n'a pas permis d'obtenir le produit attendu.

Le tableau suivant récapitule les essais qui ont été effectués en présence de 1,7% molaire de Fe₃(CO)₁₂ dans divers solvants à 65°C sous 25 bars d'hydrogène pendant 18h.

Les réactions ont été effectuées dans un autoclave de 20 mL en présence de 4mL de solvant dégazé.

Les essais ont été conduits à l'échelle de 1 mmol avec un rapport aldéhyde/amine de 1/1 avec 1% d'acide p-toluène sulfonique

**Table 2. Résultats des réactions d'amination réductrice catalysée par Fe₃(CO)₁₂**

| | Solvant | Observations |
|---|---|---|
| **1** | toluène | 0% d'ivabradine |
| **2** | dichlorométhane | 0% d'ivabradine |
| **3** | tétrahydrofurane | 0% d'ivabradine |
| **4** | N-méthylpyrrolidone | 0% d'ivabradine |

Un groupement électroattracteur est un groupement qui attire les électrons plus que ne le ferait un atome d'hydrogène qui occuperait la même position dans la molécule.

Parmi les groupements électroattracteurs on peut citer à titre non limitatif les groupements suivants : ester, acide, nitrile, aldéhyde, cétone, amide, nitro, sulfone, sulfoxyde, sulfoximine, sulfonamide ou diester phosphorique.

Dans un mode de réalisation de l'invention, le catalyseur à base de fer utilisé dans la réaction d'amination réductrice du composé de formule (V) par le composé de formule (VI) a la formule générale suivante : 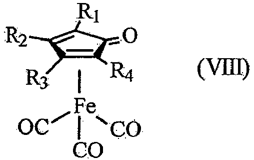 dans laquelle R₂ et R₃ représentent chacun un atome d'hydrogène ou forment ensemble avec les atomes de carbone qui les portent un carbocycle ou un hétérocycle possédant 3 à 7 chaînons, et R₁ et R₄ représentent de manière indépendante :
- soit un groupement -SiR₅R₆R₇, dans lequel R₅, R₆ et R₇, représentent de manière indépendante un groupement (C₁-C₆)alkyle linéaire ou ramifié, optionnellement substitué, ou un groupement aryle optionnellement substitué,
- soit un groupement aromatique ou hétéroaromatique, optionnellement substitué,
- soit un groupement (C₁-C₆)alkyle linéaire ou ramifié, optionnellement substitué.

Le catalyseur de formule (VIII) utilisé dans la réaction d'amination réductrice du composé de formule (V) par le composé de formule (VI) est préférentiellement choisi parmi les catalyseurs suivants :

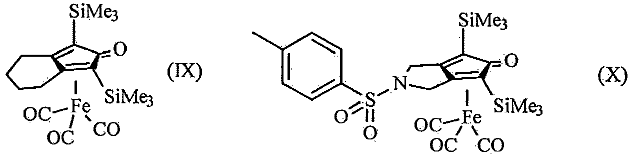

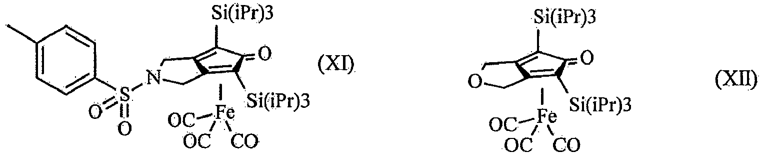

Dans un autre mode de réalisation de l'invention, le catalyseur à base de fer utilisé dans la réaction d'amination réductrice du composé de formule (V) par le composé de formule (VI) a la formule générale suivant : 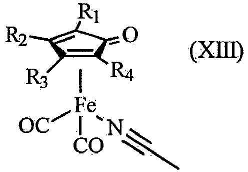 dans laquelle R₂ et R₃, représentent chacun un atome d'hydrogène ou forment ensemble avec les atomes de carbone qui les portent un carbocycle ou un hétérocycle possédant 3 à 7 chaînons, et R₁ et R₄ représentent de manière indépendante :
- soit un groupement -SiR₅R₆R₇, dans lequel R₅, R₆ et R₇, représentent de manière indépendante un groupement (C₁-C₆)alkyle linéaire ou ramifié, optionnellement substitué, ou un groupement aromatique ou hétéroaromatique optionnellement substitué,
- soit un groupement aromatique ou hétéroaromatique, optionnellement substitué,
- soit un groupement (C₁-C₆)alkyle linéaire ou ramifié, optionnellement substitué.

Le catalyseur de formule (XIII) utilisé dans la réaction d'amination réductrice du composé de formule (V) par le composé de formule (VI) est préférentiellement choisi parmi les catalyseurs suivants :

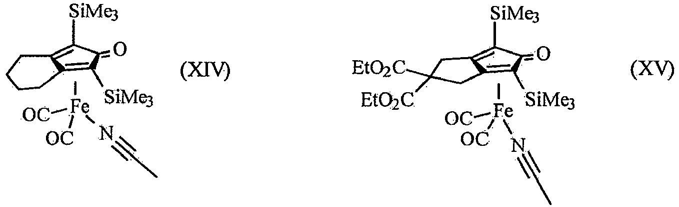

Dans un autre mode de réalisation de l'invention, le catalyseur à base de fer utilisé dans la réaction d'amination réductrice du composé de formule (V) par le composé de formule (VI) a la formule suivante :

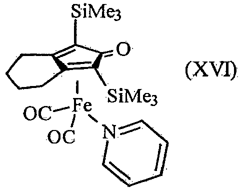

Dans un autre mode de réalisation de l'invention, le catalyseur à base de fer utilisé dans la réaction d'amination réductrice du composé de formule (V) par le composé de formule (VI) a la formule suivante :

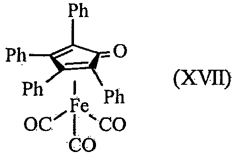

La quantité de catalyseur engagée dans la réaction d'amination réductrice du composé de formule (V) par le composé de formule (VI) est comprise entre 1% et 10% molaire par rapport à l'aldéhyde.

La quantité de *N*-oxyde de triméthylamine engagée dans la réaction d'animation réductrice du composé de formule (V) par le composé de formule (VI) est comprise entre 0 et 3 équivalents par rapport au catalyseur, plus préférentiellement entre 0,5 et 1,5 équivalent par rapport au catalyseur.

La pression de dihydrogène lors de la réaction d'amination réductrice du composé de formule (V) par le composé de formule (VI) est préférentiellement comprise entre 1 et 20 bars, plus préférentiellement entre 1 et 10 bars, encore plus préférentiellement entre 1 et 5 bars.

Parmi les solvants pouvant être utilisés pour effectuer la réaction d'amination réductrice du composé de formule (V) par le composé de formule (VI), on peut citer à titre non limitatif les alcools, préférentiellement l'éthanol, l'isopropanol, le trifluoroéthanol, le tert-butanol ou le méthanol, le tétrahydrofurane, l'acétate d'éthyle, l'acétonitrile et le dioxane.

Le solvant préférentiellement utilisé pour effectuer la réaction d'amination réductrice du composé de formule (V) par le composé de formule (VI) est l'éthanol.

La température de la réaction d'amination réductrice entre le composé de formule (V) et le composé de formule (VI) est préférentiellement comprise entre 25 et 100°C, plus préférentiellement entre 50 et 100°C, encore plus préférentiellement entre 80 et 100 °C.

Les exemples ci-dessous illustrent l'invention.

Les purifications par chromatographie sur colonne sont effectuées sur gel de silice 70-230 mesh.

Les spectres RMN ¹H sont enregistrés à 400 MHz.

Les déplacements chimiques sont exprimés en ppm (référence interne TMS).

Les abréviations suivantes ont été utilisées pour qualifier les pics : singulet (s), doublet (d), doublet de doublet (dd), triplet (t), quadruplet (q), multiplet (m).

Les catalyseurs utilisés dans le procédé de l'invention peuvent être préparés suivant les méthodes décrites dans les publications suivantes : Synlett 1992, pp1002-1004, Synlett 1993, pp924-926 et Advanced Synthesis and Catalysis 2012, 354 (4), pp597-601.

### Procédure générale A de préparation de la 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

Dans un autoclave propre et sec en acier inoxydable, on introduit sous atmosphère d'argon 1 mmol de [(7*S*)-3,4-diméthoxybicyclo[4.2.0]octa-1,3,5-trién-7-yl]-*N*-méthylméthanamine et 1 mmol de 3-(7,8-diméthoxy-2-oxo-1,2,4,5-tétrahydro-3*H*-3-benzazépin-3-yl)propanal. Le mélange est dégazé par trois cycles vide/argon et 2 mL d'éthanol distillé et dégazé sont ajoutés. La solution est agitée une heure à 85°C.

Un mélange de 5% molaire de complexe de fer et 5% molaire de *N*-oxyde de triméthylamine dans 1 mL d'éthanol est préparé pendant 30 minutes dans un tube de Schlenk sous atmosphère d'argon puis introduit dans l'autoclave.

L'autoclave est ensuite mis sous pression d'hydrogène (5 bars) et le milieu réactionnel est agité 16 heures à 85°C puis l'autoclave est ramené à température ambiante et décompressé. Le milieu réactionnel est filtré sur alumine neutre désactivée (3% d'eau) en utilisant l'acétate d'éthyle comme solvant.

Le produit brut est purifié sur gel de silice (éluant : pentane/acétate d'éthyle (95/5) avec 0,5% de triéthylamine) pour obtenir le produit attendu.

### Procédure générale B de préparation de la 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

Dans un autoclave propre et sec en acier inoxydable, on introduit sous atmosphère d'argon 1 mmol de [(7S)-3,4-diméthoxybicyclo[4.2.0]octa-1,3,5-trién-7-yl]-*N*-méthylméthanamine et 1 mmol de 3-(7,8-diméthoxy-2-oxo-1,2,4,5-tétrahydro-3*H*-3-benzazépin-3-yl)propanal. Le mélange est dégazé par trois cycles vide/argon et 3 mL d'éthanol distillé et dégazé sont ajoutés. La solution est agitée une heure à 85°C. Le complexe de fer (5mol%) est ajouté sous argon. L'autoclave est ensuite mis sous pression d'hydrogène (5 bars) et le milieu réactionnel est agité 16 heures à 85°C puis l'autoclave est ramené à température ambiante et décompressé.

Le milieu réactionnel est filtré sur alumine neutre désactivée (3% d'eau) en utilisant l'acétate d'éthyle comme solvant.

Le produit brut est purifié sur gel de silice (éluant : pentane/acétate d'éthyle (95/5) avec 0,5% de triéthylamine) pour obtenir le produit attendu.

### EXEMPLE 1

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one est préparée selon la procédure générale A en présence du catalyseur au fer de formule (IX).
Rendement = 61 %

RMN ¹H (CDCl₃) : δ = 6,67 et 6,64 (2s, 2H) ; 6,55 et 6,50 (2s, 2H) ; 3,79 et 3,78 (2s, 12H) ; 3,76 (s, 2H) ; 3, 67 (m, 2H) ; 3,45 (m, 3H) ; 3,17 (dd, 1H) ; 2,99 (m, 2H) ; 2,65 (m, 2H) ; 2,50 (dd, 1H) ; 2,37 (t, 2H) ; 2,26 (s, 3H) ; 1,72 (q, 2H).

### EXEMPLE 2

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one est préparée selon la procédure générale A en présence du catalyseur au fer de formule (X).
Rendement = 63%

### EXEMPLE 3

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one est préparée selon la procédure générale A en présence du catalyseur au fer de formule (XI).
Rendement = 79%

### EXEMPLE 4

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one est préparée selon la procédure générale A en présence du catalyseur au fer de formule (XII).
Rendement = 68%

### EXEMPLE 5

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one est préparée selon la procédure générale B en présence du catalyseur au fer de formule (XIV).
Rendement = 68%

### EXEMPLE 6

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H-*3-benzazépin-2-one est préparée selon la procédure générale B en présence du catalyseur au fer de formule (XV).
Rendement = 68%

### EXEMPLE 7

La 3-{3-[{[(7S)-3,4-diméthoxybicyclo[4.2.0]octa-1,3,5-trién-7-yl]méthyl}(methyl)amino] propyl}-7,8-diméthoxy-1,3,4,5-tétrahydro-2*H-*3-benzazépin-2-one est préparée selon la procédure générale B en présence du catalyseur au fer de formule (XVI).
Rendement = 59%

### EXEMPLE 8

La 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one est préparée selon la procédure générale B en présence du catalyseur au fer de formule (XVII). Rendement = 48%

## Revendications

1. Procédé de synthèse de l'ivabradine de formule (I) : 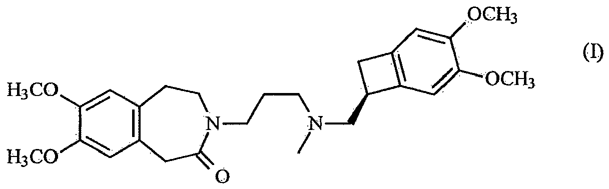 **caractérisé en ce que** l'on soumet le composé de formule (V) : 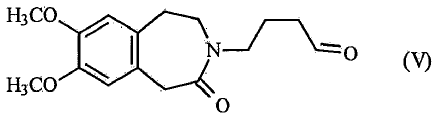 à une réaction d'amination réductrice par l'amine de formule (VI) : 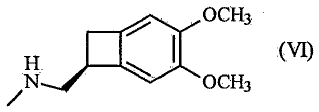 en présence d'un catalyseur à base de fer de formule (VII) : 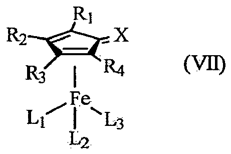 dans laquelle R₁, R₂, R₃ et R₄ représentent de manière indépendante :
• un atome d'hydrogène, ou
• un groupement -SiR₅R₆R₇, dans le quel R₅, R₆ et R₇, représentent de manière indépendante un groupement (C₁-C₆)alkyle linéaire ou ramifié, optionnellement substitué, ou un groupement aromatique ou hétéroaromatique optionnellement substitué, ou
• un groupement aromatique ou hétéroaromatique, optionnellement substitué, ou
• un groupement (C₁-C₆)alkyle linéaire ou ramifié, optionnellement substitué, ou
• un groupement électroattracteur, ou
• une amine aliphatique, aromatique, hétéroaromatique ou portant un groupement électroattracteur, ou
• un éther aliphatique ou aromatique, hétéroaromatique,
ou R₁ et R₂, ou R₂ et R₃, ou R₃ et R₄ forment deux à deux avec les atomes de carbone qui les portent un carbocycle ou un hétérocycle possédant 3 à 7 chaînons,
X représente :
• un atome d'oxygène, ou
• un groupement -NH, ou un atome d'azote substitué par un groupement aliphatique, aromatique, hétéroaromatique ou électroattracteur, ou
• un groupement -PH, ou un atome de phosphore substitué par un ou plusieurs groupements aliphatiques, aromatiques ou électroattracteurs, ou
• un atome de soufre,
L₁, L₂ et L₃ représentent de manière indépendante un groupement carbonyle, nitrile, isonitrile, hétéroaromatique, phosphine, phosphite, phosphonite, phosphoramidite, phosphinite, phospholane, phospholène, amine aliphatique, amine aromatique, amine hétéroaromatique, amine portant un groupement électroattracteur, éther aliphatique, éther aromatique, éther hétéroaromatique, sulfone, sulfoxyde, sulfoximine, ou un carbène *N*-hétérocyclique possédant l'une des deux formules suivantes : 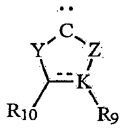 dans laquelle Y et Z représentent de manière indépendante un atome de soufre ou d'oxygène, ou un groupement NR₈, dans lequel R₈ représente un groupement alkyle optionnellement substitué, un groupement aromatique ou hétéroaromatique optionnellement substitué,
K représente un atome de carbone ou d'azote,
R₉, R₁₀ représentent de manière indépendante un atome d'hydrogène, un groupement alkyle optionnellement substitué, un groupement aromatique ou hétéroaromatique optionnellement substitué, un atome d'halogène, un éther aliphatique, aromatique ou hétéroaromatique, une amine aliphatique aromatique ou hétéroaromatique,
ou R₉ et R₁₀ forment deux à deux avec les atomes qui les portent un carbocycle ou un hétérocycle possédant 3 à 7 chaînons,
ou 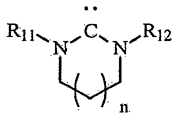 dans laquelle R₁₁ et R₁₂ représentent de manière indépendante un groupement alkyle optionnellement substitué, un groupement aromatique ou hétéroaromatique optionnellement substitué et n est égal à 1 ou 2,
en présence ou non de N-oxyde de triméthylamine,
sous une pression de dihydrogène comprise entre 1 et 20 bars.
dans un solvant organique ou un mélange de solvants organiques,
à une température comprise entre 25 et 100°C.

2. Procédé de synthèse selon la revendication 1, **caractérisé en ce que** le catalyseur à base de fer possède la formule générale suivante : 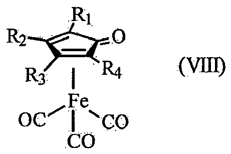 dans laquelle R₂ et R₃ représentent chacun un atome d'hydrogène ou forment ensemble avec les atomes de carbone qui les portent un carbocycle ou un hétérocycle possédant 3 à 7 chaînons,
et R₁ et R₄ représentent de manière indépendante :
• soit un groupement -SiR₅R₆R₇, dans lequel R₅, R₆ et R₇ représentent de manière indépendante un groupement (C₁-C₆)alkyle linéaire ou ramifié, optionnellement substitué, ou un groupement aryle optionnellement substitué,
• soit un groupement aromatique ou hétéroaromatique, optionnellement substitué,
• soit un groupement (C₁-C₆)alkyle linéaire ou ramifié, optionnellement substitué.

3. Procédé de synthèse selon la revendication 2, **caractérisé en ce que** le catalyseur à base de fer est choisi parmi :

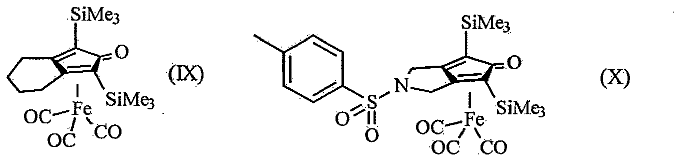

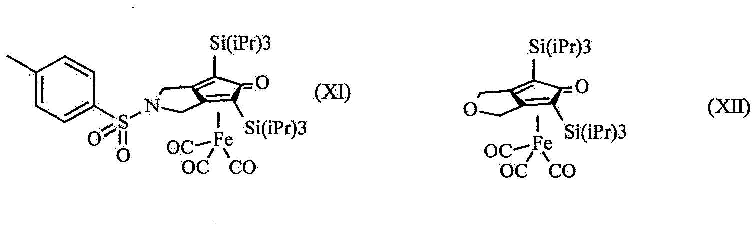

4. Procédé de synthèse selon la revendication 1, **caractérisé en ce que** le catalyseur à base de fer possède la formule générale suivante : 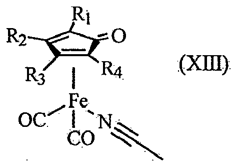 dans laquelle R₂ et R₃ représentent chacun un atome d'hydrogène ou forment ensemble avec les atomes de carbone qui les portent un carbocycle ou un hétérocycle possédant 3 à 7 chaînons,
et R₁ et R₄ représentent de manière indépendante :
• soit un groupement -SiR₅R₆R₇, dans lequel R₅, R₆ et R₇ représentent de manière indépendante un groupement (C₁-C₆)alkyle linéaire ou ramifié, optionnellement substitué, ou un groupement aromatique ou hétéroaromatique optionnellement substitué,
• soit un groupement aromatique ou hétéroaromatique, optionnellement substitué,
• soit un groupement (C₁-C₆)alkyle linéaire ou ramifié, optionnellement substitué.

5. Procédé de synthèse selon la revendication 4, **caractérisé en ce que** le catalyseur à base de fer est choisi parmi :

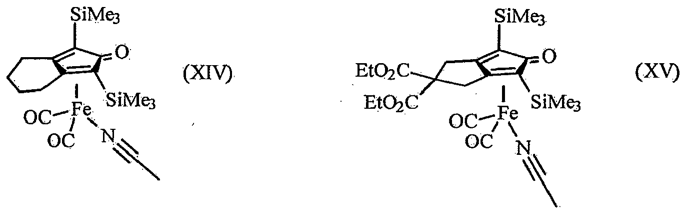

6. Procédé selon la revendication 1, **caractérisé en ce que** le catalyseur à base de fer a la formule suivante :

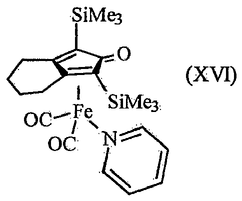

7. Procédé selon la revendication 1, **caractérisé en ce que** le catalyseur à base de fer a la formule suivante :

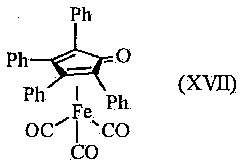

8. Procédé de synthèse selon l'une quelconque des revendications 1 à 7, **caractérisé en ce que** la quantité de catalyseur engagée dans la réaction d'amination réductrice est comprise entre 1 % et 10% molaire par rapport à l'aldéhyde.

9. Procédé de synthèse selon l'une quelconque des revendications 1 à 8, **caractérisé en ce que** la quantité de *N*-oxyde de triméthylamine engagée dans la réaction d'amination réductrice est comprise entre 0 et 3 équivalents par rapport au catalyseur.

10. Procédé de synthèse selon la revendication 9, **caractérisé en ce que** la quantité de *N*-oxyde de triméthylamine engagée dans la réaction d'amination réductrice est comprise entre 0,5 et 1,5 équivalent par rapport au catalyseur.

11. Procédé de synthèse selon l'une quelconque des revendications 1 à 10, **caractérisé en ce que** la pression de dihydrogène lors de la réaction d'amination réductrice est comprise entre 1 et 10 bars.

12. Procédé de synthèse selon l'une quelconque des revendications 1 à 11, **caractérisé en ce que** le solvant de la réaction d'amination réductrice est un alcool.

13. Procédé de synthèse selon la revendication 12, **caractérisé en ce que** le solvant de la réaction d'amination réductrice est l'éthanol.

14. Procédé de synthèse selon l'une quelconque des revendications 1 à 13, **caractérisé en ce que** la température de la réaction d'amination réductrice est comprise entre 50 et 100°C.

## Patentansprüche

1. Verfahren zur Synthese von Ivabradin der Formel (I): 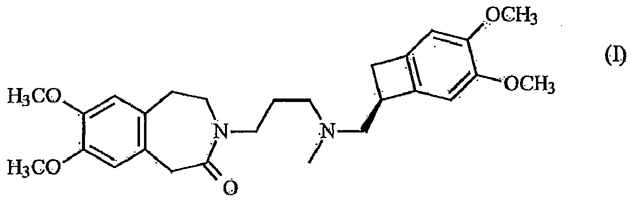 **dadurch gekennzeichnet, dass** man die Verbindung der Formel (V): 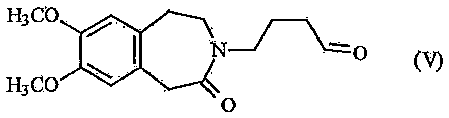 einer reduzierenden Aminierungsreaktion mit dem Amin der Formel (VI): 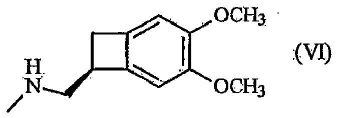 unterwirft,
in Gegenwart eines Katalysators auf Eisengrundlage der Formel (VII): 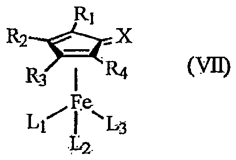 in der R₁, R₂, R₃ und R₄ unabhängig voneinander:
• ein Wasserstoffatom oder
• eine Gruppe -SiR₅R₆R₇, worin R₅, R₆ und R₇ unabhängig voneinander eine geradkettige oder verzweigte, gegebenenfalls substituierte (C₁-C₆)-Alkylgruppe oder eine gegebenenfalls substituierte aromatische oder heteroaromatische Gruppe darstellen, oder
• eine gegebenenfalls substituierte aromatische oder heteroaromatische Gruppe, oder
• eine geradkettige oder verzweigte, gegebenenfalls substituierte (C₁-C₆)-Alkylgruppe, oder
• eine Elektronen anziehende Gruppe, oder
• ein aliphatisches, aromatisches, heteroaromatisches oder eine Elektronen anziehende Gruppe tragendes Amin, oder
• einen aliphatischen, aromatischen oder heteroaromatischen Ether bedeuten,
oder R₁ und R₂ oder R₂ und R₃ oder R₃ und R₄ jeweils zu zweit mit den sie tragenden Kohlenstoffatomen einen Carbocyclus oder Heterocyclus mit 3 bis 7 Kettengliedern bilden,
X:
• ein Sauerstoffatom, oder
• eine Gruppe -NH oder ein durch eine aliphatische, aromatische, heteroaromatische oder Elektronen anziehende Gruppe substituiertes Stickstoffatom, oder
• eine Gruppe -PH oder ein durch eine oder mehrere aliphatische, aromatische, heteroaromatische oder Elektronen anziehende Gruppen substituiertes Phosphoratom, oder
• ein Schwefelatom
bedeutet,
L₁, L₂ und L₃ unabhängig voneinander eine Carbonyl-, Nitril-, Isonitril-, heteroaromatische, Phosphin-, Phosphit-, Phosphonit-, Phosphoramidit-, Phosphinit-, Phospholan-, Phospholen-, aliphatische Amin-, aromatische Amin-, heteroaromatische Amin-, eine Elektronen anziehende Gruppe aufweisende Amin-, aliphatische Ether-, aromatische Ether-, heteroaromatische Ether-, Sulfon-, Sulfoxid-, Sulfoximin-gruppe oder ein *N-*heterocyclisches Carben bedeuten, welches eine der folgenden beiden Formeln aufweist: 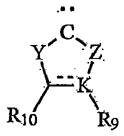 in der Y und Z unabhängig voneinander ein Schwefel- oder Sauerstoffatom oder eine Gruppe NR₈ darstellen, worin R₈ eine gegebenenfalls substituierte Alkylgruppe, eine gegebenenfalls substituierte aromatische oder heteroaromatische Gruppe darstellt,
K ein Kohlenstoff- oder Stickstoffatom bedeutet,
R₉ und R₁₀ unabhängig voneinander ein Wasserstoffatom oder eine gegebenenfalls substituierte Alkylgruppe, gegebenenfalls substituierte aromatische oder heteroaromatische Gruppe, ein Halogenatom, einen aliphatischen, aromatischen oder heteroaromatischen Ether, ein aliphatisches, aromatisches oder heteroaromatisches Amin bedeuten, oder R₉ und R₁₀ jeweils zu zweit mit den sie tragenden Atomen einen Carbocyclus oder Heterocyclus mit 3 bis 7 Kettengliedern bilden,
oder 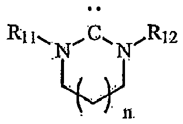 in der R₁₁ und R₁₂ unabhängig voneinander eine gegebenenfalls substituierte Alkylgruppe, eine gegebenenfalls substituierte aromatische oder heteroaromatische Gruppe bedeuten und n 1 oder 2 darstellt,
in Gegenwart oder in Abwesenheit von *N*-Trimethylaminoxid,
bei einem Wasserstoffdruck zwischen 1 und 20 bar,
in einem organischen Lösungsmittel oder einer Mischung von organischen Lösungsmitteln,
bei einer Temperatur zwischen 25 und 100 °C.

2. Syntheseverfahren nach Anspruch 1, **dadurch gekennzeichnet, dass** der Katalysator auf Eisengrundlage der folgenden allgemeinen Formel entspricht: 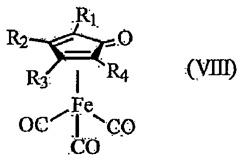 in der R₂ und R₃ jeweils ein Wasserstoffatom bedeuten oder gemeinsam mit den sie tragenden Kohlenstoffatomen einen Carbocyclus oder Heterocyclus mit 3 bis 7 Kettengliedern bilden,
und R₁ und R₄ unabhängig voneinander:
• entweder eine Gruppe -SiR₅R₆R₇, worin R₅, R₆ und R₇ unabhängig voneinander eine geradkettige oder verzweigte, gegebenenfalls substituierte (C₁-C₆)-Alkylgruppe oder eine gegebenenfalls substituierte Arylgruppe bedeuten,
• oder eine gegebenenfalls substituierte aromatische oder heteroaromatische Gruppe,
• oder eine geradkettige oder verzweigte, gegebenenfalls substituierte (C₁-C₆)-Alkylgruppe bedeuten.

3. Syntheseverfahren nach Anspruch 2, **dadurch gekennzeichnet, dass** der Katalysator auf Eisengrundlage ausgewählt ist aus:

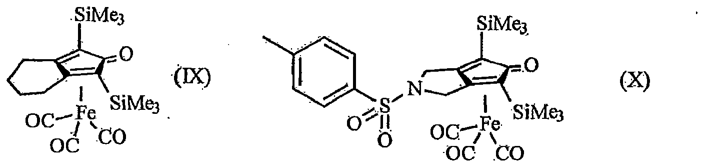

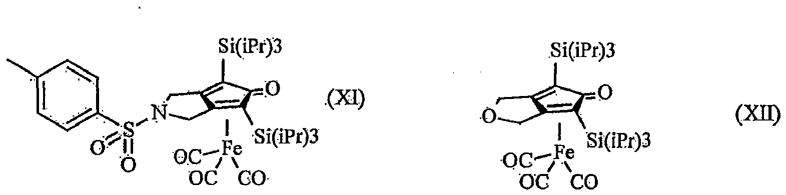

4. Syntheseverfahren nach Anspruch 1, **dadurch gekennzeichnet, dass** der Katalysator auf Eisengrundlage der folgenden allgemeinen Formel entspricht: 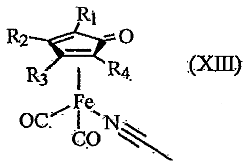 in der R₂ und R₃ jeweils ein Wasserstoffatom bedeuten oder gemeinsam mit den sie tragenden Kohlenstoffatomen einen Carbocyclus oder Heterocyclus mit 3 bis 7 Kettengliedern bilden
und R₁ und R₄ unabhängig voneinander:
• entweder eine Gruppe -SiR₅R₆R₇, worin R₅, R₆ und R₇ unabhängig voneinander eine geradkettige oder verzweigte, gegebenenfalls substituierte (C₁-C₆)-Alkylgruppe oder eine gegebenenfalls substituierte aromatische oder heteroaromatische Gruppe bedeuten,
• oder eine gegebenenfalls substituierte aromatische oder heteroaromatische Gruppe,
• oder eine geradkettige oder verzweigte, gegebenenfalls substituierte (C₁-C₆)-Alkylgruppe bedeuten.

5. Syntheseverfahren nach Anspruch 4, **dadurch gekennzeichnet, dass** der Katalysator auf Eisengrundlage ausgewählt wird aus:

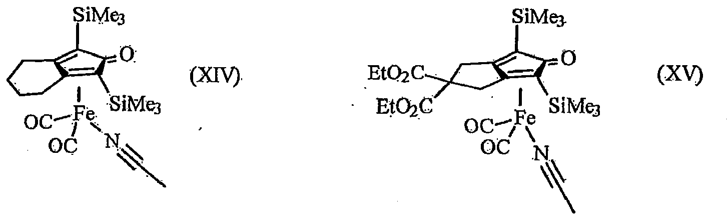

6. Verfahren nach Anspruch 1, **dadurch gekennzeichnet, dass** der Katalysator auf Eisengrundlage der folgenden Formel entspricht:

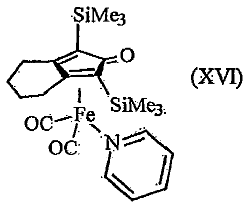

7. Verfahren nach Anspruch 1, **dadurch gekennzeichnet, dass** der Katalysator auf Eisengrundlage der folgenden Formel entspricht:

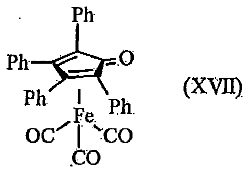

8. Syntheseverfahren nach einem der Ansprüche 1 bis 7, **dadurch gekennzeichnet, dass** die bei der reduzierenden Aminierungsreaktion eingesetzte Menge des Katalysators zwischen 1 und 10 Mol-% bezogen auf den Aldehyd beträgt.

9. Syntheseverfahren nach einem der Ansprüche 1 bis 8, **dadurch gekennzeichnet, dass** die bei der reduzierenden Aminierungsreaktion eingesetzte Menge N-Trimethylaminoxid zwischen 0 und 3 Äquivalente bezogen auf den Katalysator beträgt.

10. Syntheseverfahren nach Anspruch 9, **dadurch gekennzeichnet, dass** die bei der reduzierenden Aminierungsreaktion eingesetzte Menge des *N*-Trimethylaminoxids zwischen 0,5 und 1,5 Äquivalente bezogen auf den Katalysator beträgt.

11. Syntheseverfahren nach einem der Ansprüche 1 bis 10, **dadurch gekennzeichnet, dass** der Wasserstoffdruck bei der reduzierenden Aminierungsreaktion zwischen 1 und 10 bar beträgt.

12. Syntheseverfahren nach einem der Ansprüche 1 bis 11, **dadurch gekennzeichnet, dass** das Lösungsmittel der reduzierenden Aminierungsreaktion ein Alkohol ist.

13. Syntheseverfahren nach Anspruch 12, **dadurch gekennzeichnet, dass** das Lösungsmittel der reduzierenden Aminierungsreaktion Ethanol ist.

14. Syntheseverfahren nach einem der Ansprüche 1 bis 13, **dadurch gekennzeichnet, dass** die Temperatur der reduzierenden Aminierungsreaktion zwischen 50 und 100 °C beträgt.

## Claims

1. Process for the synthesis of ivabradine of formula (I): 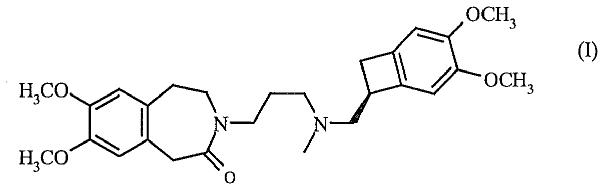 **characterised in that** the compound of formula (V): 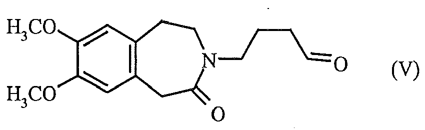 is subjected to a reductive amination reaction with the amine of formula (VI): 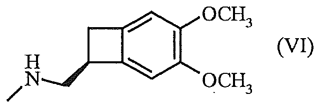 in the presence of an iron-based catalyst of formula (VII): 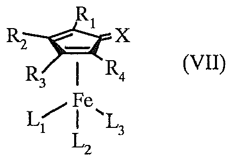 wherein R₁, R₂, R₃ and R₄ independently represent:
• a hydrogen atom, or
• a group -SiR₅R₆R₇, wherein R₅, R₆ and R₇ independently represent an optionally substituted, linear or branched (C₁-C₆)alkyl group or an optionally substituted aromatic or heteroaromatic group, or
• an optionally substituted aromatic or heteroaromatic group, or
• an optionally substituted, linear or branched (C₁-C₆)alkyl group, or
• an electron-attracting group, or
• an amine group that is aliphatic, aromatic, heteroaromatic or carrying an electron-attracting group, or
• an aliphatic, aromatic or heteroaromatic ether group,
or the pairs R₁ and R₂, or R₂ and R₃, or R₃ and R₄ form, together with the carbon atoms carrying them, a 3- to 7-membered carbocycle or heterocycle,
X represents:
• an oxygen atom, or
• an -NH group, or a nitrogen atom which is substituted by an aliphatic, aromatic, heteroaromatic or electron-attracting group, or
• a -PH group, or a phosphorus atom which is substituted by one or more aliphatic, aromatic or electron-attracting groups, or
• a sulphur atom,
L₁, L₂ and L₃ independently represent a carbonyl, nitrile, isonitrile, heteroaromatic, phosphine, phosphite, phosphonite, phosphoramidite, phosphinite, phospholane, phospholene, aliphatic amine, aromatic amine, heteroaromatic amine, electron-attracting-group-carrying amine, aliphatic ether, aromatic ether, heteroaromatic ether, sulphone, sulphoxide or sulphoximine group or an *N*-heterocyclic carbene group having one of the two following formulae: 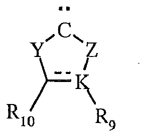 wherein Y and Z independently represent a sulphur or oxygen atom or a group NR₈ wherein R₈ represents an optionally substituted alkyl group or an optionally substituted aromatic or heteroaromatic group,
K represents a carbon or nitrogen atom,
R₉ and R₁₀ independently represent a hydrogen atom, an optionally substituted alkyl group, an optionally substituted aromatic or heteroaromatic group, a halogen atom, an aliphatic, aromatic or heteroaromatic ether group, an aliphatic, aromatic or heteroaromatic amine group, or the pair R₉ and R₁₀ form, together with the atoms carrying them, a 3- to 7-membered carbocycle or heterocycle,
or 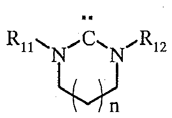 wherein R₁₁ and R₁₂ independently represent an optionally substituted alkyl group or an optionally substituted aromatic or heteroaromatic group, and n is 1 or 2,
in the presence or not of trimethylamine *N*-oxide,
under dihydrogen pressure of from 1 to 20 bars,
in an organic solvent or mixture of organic solvents,
at a temperature from 25 to 100°C.

2. Synthesis process according to claim 1, **characterised in that** the iron-based catalyst has the following general formula: 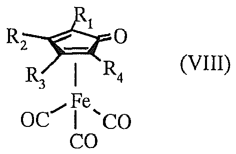 wherein R₂ and R₃ each represent a hydrogen atom or form, together with the carbon atoms carrying them, a 3- to 7-membered carbocycle or heterocycle,
and R₁ and R₄ independently represent:
• either a group -SiR₅R₆R₇, wherein R₅, R₆ and R₇ independently represent an optionally substituted, linear or branched (C₁-C₆)alkyl group or an optionally substituted aryl group,
• or an optionally substituted aromatic or heteroaromatic group,
• or an optionally substituted, linear or branched (C₁-C₆)alkyl group.

3. Synthesis process according to claim 2, **characterised in that** the iron-based catalyst is selected from:

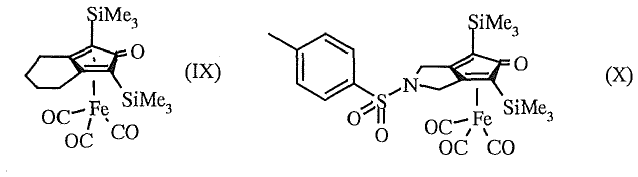

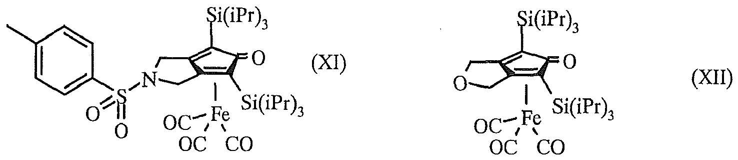

4. Synthesis process according to claim 1, **characterised in that** the iron-based catalyst has the following general formula: 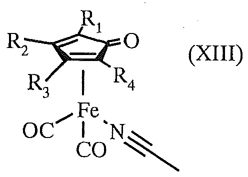 wherein R₂ and R₃ each represent a hydrogen atom or form, together with the carbon atoms carrying them, a 3- to 7-membered carbocycle or heterocycle,
and R₁ and R₄ independently represent:
• either a group -SiR₅R₆R₇, wherein R₅, R₆ and R₇ independently represent an optionally substituted, linear or branched (C₁-C₆)alkyl group or an optionally substituted aromatic or heteroaromatic group,
• or an optionally substituted aromatic or heteroaromatic group,
• or an optionally substituted, linear or branched (C₁-C₆)alkyl group.

5. Synthesis process according to claim 4, **characterised in that** the iron-based catalyst is selected from:

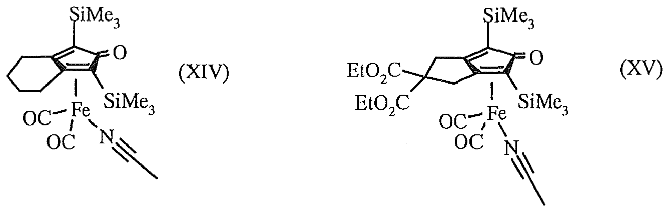

6. Process according to claim 1, **characterised in that** the iron-based catalyst has the following formula:

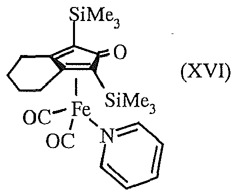

7. Process according to claim 1, **characterised in that** the iron-based catalyst has the following formula:

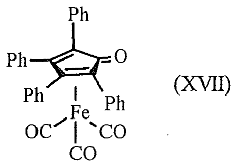

8. Synthesis process according to any one of claims 1 to 7, **characterised in that** the amount of catalyst used in the reductive amination reaction is from 1 mol% to 10 mol% relative to the aldehyde.

9. Synthesis process according to any one of claims 1 to 8, **characterised in that** the amount of trimethylamine *N*-oxide used in the reductive amination reaction is from 0 to 3 equivalents relative to the catalyst.

10. Synthesis process according to claim 9, **characterised in that** the amount of trimethylamine N-oxide used in the reductive amination reaction is from 0.5 to 1.5 equivalents relative to the catalyst.

11. Synthesis process according to any one of claims 1 to 10, **characterised in that** the dihydrogen pressure in the reductive amination reaction is from 1 to 10 bars.

12. Synthesis process according to any one of claims 1 to 11, **characterised in that** the solvent in the reductive amination reaction is an alcohol.

13. Synthesis process according to claim 12, **characterised in that** the solvent in the reductive amination reaction is ethanol.

14. Synthesis process according to any one of claims 1 to 13, **characterised in that** the temperature of the reductive amination reaction is from 50 to 100°C.
